# EUROPEAN PATENT SPECIFICATION

(11) **EP 1 136 097 B1**
(45) Date of publication and mention of the grant of the patent: **26.11.2003**
(21) Application number: 01400186.1
(22) Date of filing: 23.01.2001
(51) Int. Cl.: A61N 1/36

(54) **Living body stimulating apparatus**
Reizvorrichtung für lebendige Körper
Stimulateur de corps vivant

(30) Priority: 17.03.2000 JP 2000076884
(43) Date of publication of application: 26.09.2001
(73) Proprietor: Techno Link Co., Ltd., Niitsu-shi, Niigata-ken (JP)
(72) Inventor: KOBAYASHI, Tatsuyuki, Niigata-shi. Niigata-ken (JP)
(74) Representative: Maillet, Alain

(56) References cited:
- US-A- 3 294 092
- US-A- 4 938 223
- US-A- 5 018 524
- US-A- 5 097 833
- US-A- 5 330 515
- US-A- 5 397 338
- US-A- 5 836 081

## Description

The present invention relates to a living body stimulating apparatus, such as a low-frequency electro-therapeutic device. More particularly, the present invention concerns a stimulating apparatus of the kind comprising a conductor element with a built-in electrode which can be attached to a living body. Stimulation is achieved by an electric current flowing through the electrode into the living body.

In general, such a living body stimulating apparatus is used for the treatment of a nerve function in a diseased part, by allowing a low frequency pulse current to flow in the diseased part, said low frequency pulse current being output from a transmitter to an electrode. In Japanese Patent Un-Examined Publication No.1-146562, for example, is disclosed a living body stimulating apparatus which enables the control of the speed and intensity of stimulation, by switching stimulation signals output from an output circuit to a living body (or human body). The stimulation signals are either DC intermittent pulses for periodically outputting positive pulses, AC intermittent pulses for periodically outputting rectangular wave pulse groups consisting of positive pulses and negative pulses, alternate intermittent pulses for periodically and alternately outputting positive pulses and negative pulses, the period or amplitude of which can be varied. Document US-A-5 097 833 shows the features defined in the preamble of claim 1.

However, it is generally difficult to make pass a DC current through a human body, since it has a resistance of about 100kΩ, depending upon voltage, whilst a highfrequency AC current flows easily through a human body. For example, a human body exhibits a resistance of about 1kΩ under an AC voltage of 1kHz, said resistance being reduced by half if the frequency doubles. In other words, a human body has a capacitive impedance, and thus the in-vivo resistance tends to decrease as frequency increases. On the other hand, as the stimulation of the human body is concerned, low frequencies close to direct current, and rectangular waves comprising a high DC component are more stimulative. Thus, for the same frequency, sinusoidal waves will induce softer stimuli than rectangular waves.

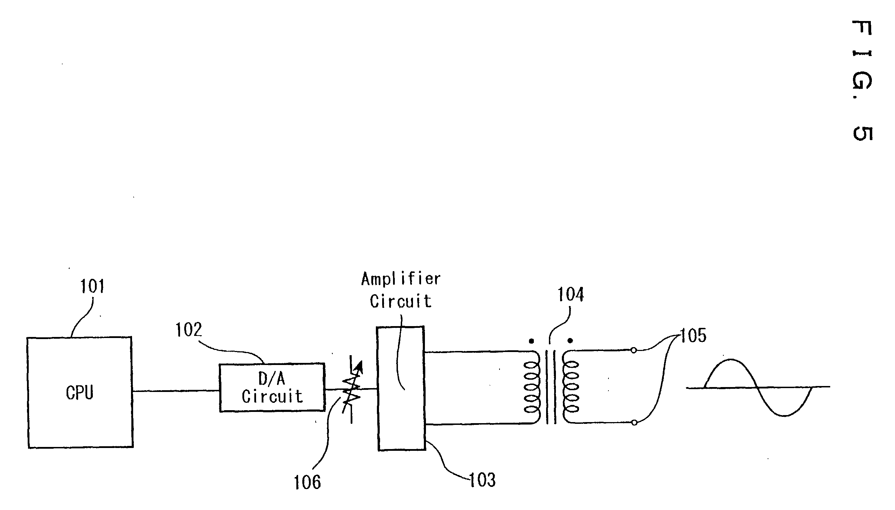

Fig.5 illustrates an example of a conventional living body stimulating apparatus for applying stimulation signals having the form of sine waves to a human body. As shown, numeral 101 designates a CPU (central processing unit) as controlling means. The CPU outputs digital data signals which are converted into analogue data signals by the D/A converter 102. Then, the analogue data signals are amplified by an amplifier 103, to provide sine-wave stimulation signals between conductive elements or output electrodes 105, over a transformer 104. The amplitude of the sine wave can be increased or decreased by operating a variable gain element 106 at the input of the amplifier 103.

Although a sinusoidal wave induces a soft stimulation of the human body, it comprises only one frequency component, which leads to a very limited therapeutic effect. Furthermore, the output circuit of such a conventional apparatus requires analogue circuits such as the D/A converter 102 and the amplifier circuit 103 which are necessary for obtaining a substantially sinusoidal waveform. The number of components is relatively high and the structure of the circuit is complex, thus leading to low power efficiency. In other words, conventional circuits outputting sinusoidal waves would require dozens of components such as transistors, resistors and capacitors.

It is a main object of the invention to solve the problems recited above and to propose a living body stimulating apparatus which provides extensive therapeutic effects while soft stimuli are applied to the living body.

To attain the above objects, there is proposed a living body stimulating apparatus as defined in the appended claims

Other objects, features and advantages of the invention will be apparent to those skilled in the art from the following description of the various embodiments of the invention, wherein reference is made to the accompanying drawings, of which:
Fig.1 is a circuit diagram showing a living body stimulating apparatus in accordance with a first embodiment of the invention;
Fig.2 is a waveform diagram showing waveforms at several points in the apparatus of Fig.1;
Fig.3 is a waveform diagram of a stimulating signal when a dummy resistance is connected between the output electrodes of the apparatus;
Fig.4 is another waveform diagram of a stimulating signal when the output electrodes of the apparatus are applied onto a human waist;
Fig.5 is a circuit diagram showing a conventional living body stimulating apparatus.

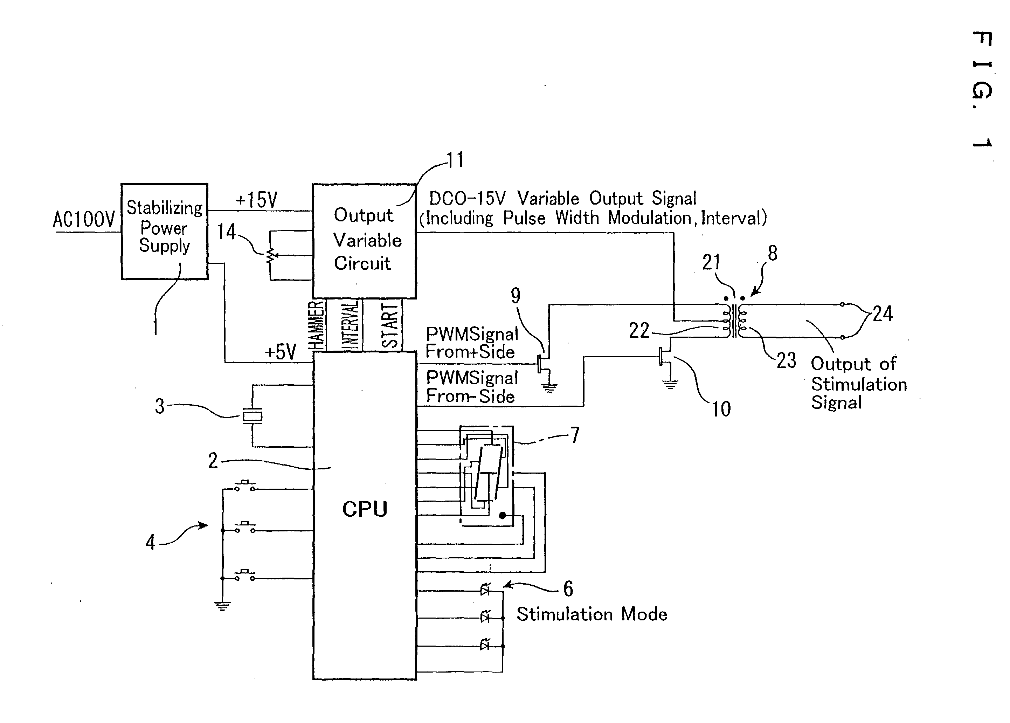

First, an overall structure of an apparatus of the invention will be described with reference to Fig.1. Reference numeral 1 designates a stabilized power supply unit connected to an AC source and providing a DC stable voltage. In the embodiment, AC source voltage of AC 100 V is converted into DC voltages of DC+15V and DC+5V, respectively. Reference numeral 2 designates a CPU (central processing unit) which serves as a control means powered by said DC+5V from the said stabilized power supply unit 1. The CPU inputs reference clock signals from a crystal oscillator 3. As well known in the art, CPU 2 is integrated with an input/output means, a processing means, a memory means in which a control sequence is stored, said control sequence being able to generate predetermined patterns of stimulating electric signals.

To an input port of said CPU 2 are connected a plurality of switches 4 for selecting a specific stimulation mode from among several stimulation modes. A plurality of LEDs 6 are connected to an output port of said CPU 2 for displaying which stimulation mode is currently active. Furthermore, to the output port of the CPU 2 are connected a segment LED 7 as time display means for counting and displaying the stimulation time, two FETs 9, 10 included in the stimulus signal generating means 8, and an output-variable circuit 11 for varying the amplitude and interval (pause) of stimulating signals to be applied to a living body. For the sake of simplicity, only one segment LED 7 is illustrated in the present embodiment, but two or more segment LEDs 7 could be connected in parallel. Alternatively, the foregoing LEDs 6 and the segment LED 7 may be integrated together in a common LCD display.

The output-variable circuit 11 is powered by the DC voltage of DC+15V provided by the stabilized power supply unit 1 and receives control signals from the CPU 2, namely the strong stimulation control signal (HAMMER), the stimulation pause setting signal (INTERVAL), and the stimulation start signal (START). The circuit 11 outputs a variable output signal. A manually operable variable resistor 14 is provided for setting the level of the variable output signal. The variable output signal is supplied to the stimulus generator means 8. This signal has a rectangular waveform of predetermined recurrence frequency and its amplitude is modulated in the range DC 0V to DC+15V.

The stimulus generator means 8 subjects the variable output signal from the output-variable circuit 11 to pulse width modulation. The stimulus generator comprises FETs 9, 10 serving as switching means and a transformer 21, the primary and secondary sides of which are insulated from each other. More specifically, the primary winding 22 of the transformer 21 has a center tap connected to the variable output signal line of the said output-variable circuit 11, while the secondary winding 23 have ends connected to a pair of output electrodes 24 serving as conductive elements. The drain of the grounded source FET 9 is connected to one end of the primary winding 22 of the transformer 21, while the drain of the other source grounded FET 10 is connected to the other end of the primary winding 22 . The + side PWM signals from the CPU 2 are supplied to the gate of the FET 9, while - side PWM signals from the CPU 2 are supplied to a gate of the FET 10.

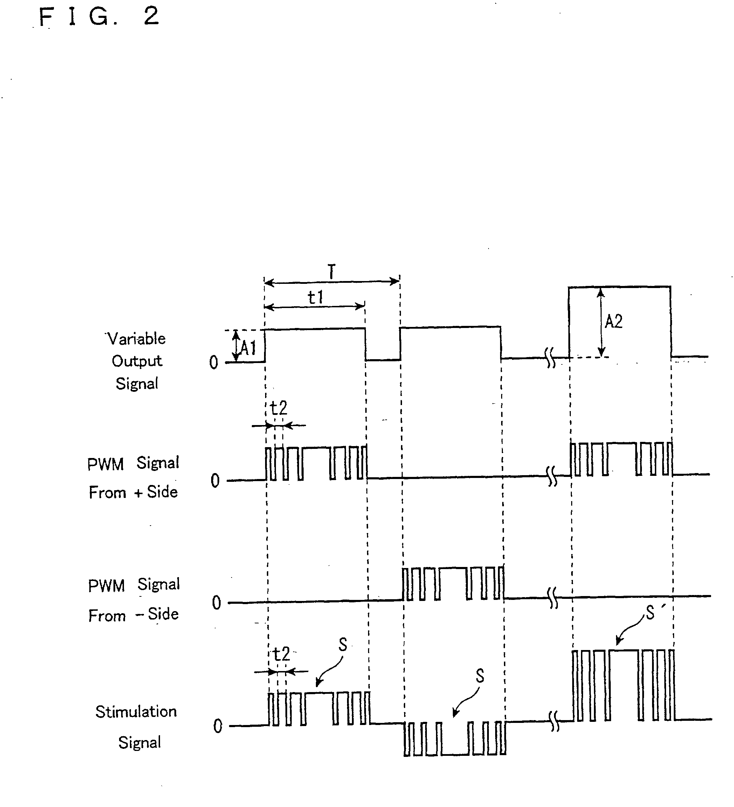

Next, the action of the above-structured apparatus will be explained with reference to the waveform diagram of Fig.2, in which the uppermost waveform shows a variable output signal from the output-variable circuit 11, followed by the respective waveforms of the + side PWM signals, - side PWM signals and the stimulation signals between the output electrodes 24.

If a specific stimulation mode is selected by the switch 4, and then a start switch (not shown) is operated, the LED 6 corresponding to the specific stimulation mode selected is switched on by the CPU 2. The CPU 2 controls also the stimulus generator means 8 and the output-variable circuit 11 in order that the stimulation signals corresponding to the selected stimulation mode are output between the electrodes 24. The stimulation start signal is supplied from the CPU 2 to the output-variable circuit 11, and the variable-output signal is supplied from the output-variable circuit 11 to the stimulus generator means 8. As shown in Fig. 2, the variable-output signal comprises rectangular pulses defined by an amplitude A1 and a time width t1, repeating at a period T. The amplitude A1 can be varied by operating the variable resistor 14 in the range DC0V to DC+15V and thus it is possible for a user to change the degree of stimulation to a desired level. Alternatively, the period T and time width t1 can be varied by the CPU 2 (not shown in the drawing). In such an embodiment a user may obtain a more desirable stimulus by varying a parameter of the control program inside the CPU 2.

Every time a rectangular wave pulse is output from said output-variable circuit 11, the CPU 2 is enabled to output on-pulses to the FET 9 or the FET 10, during the output period of the rectangular pulse. The on-pulses have a higher frequency component than the rectangular pulse. The time width t2 of each on-pulse to the FET 9 or FET 10 gradually increases from the rising edge of said rectangular pulse until about the middle of said rectangular wave pulse and then gradually decreases until the falling edge of said rectangular pulse.

When the +side PWM signal is supplied from the CPU 2 to the FET 9 while a rectangular pulse is output to the center tap of the primary winding 22 of the transformer 21, the FET 9 is turned on during each on-pulse, so that the primary side (i.e. dotted side) of the primary winding 22 is grounded, thus inducing a voltage in the primary side (dotted side) of the secondary winding 23. Likewise, when the -side PWM signal is supplied from the CPU 2 to the FET 10 while a rectangular wave pulse is output to the center tap of the primary winding 22 of the transformer 21, the FET 10 is turned on during the output period of the on-pulse, so that the secondary side (i.e., non-dotted side) of the primary winding 22 is grounded and thus inducing a voltage in the secondary side (non-dotted side) of the secondary winding 23. Accordingly, as shown in Fig.2, the supply of the on-pulses to the gate of the FET 9 during the output period of the rectangular wave pulses allows a positive stimulation signal to be output, while the supply of the on-pulses to the gate of FET 10 during the output period of a rectangular wave pulse allows a negative stimulation signal to be output.

Thus, between the output electrodes 24 are repeatedly generated stimulation signals having a voltage level proportional to the amplitude A1, said signals being obtained by subjecting the rectangular wave pulses of the variable-output signals to pulse width modulation (PWM) through the FETs 9, 10. These stimulation signals exhibits a group S of pulses, generated at every period T, the group having the time width t1, said pulse group S alternately having a positive voltage and a negative voltage . The time width t2 of each on-pulse gradually increases from the beginning of the pulse group until the middle thereof and then gradually decreases until the group S ends. As long as a stimulation signal is output, a built-in timer (not shown) inside the CPU 2 is allowed to count time, and displays the time on the segment LED 7.

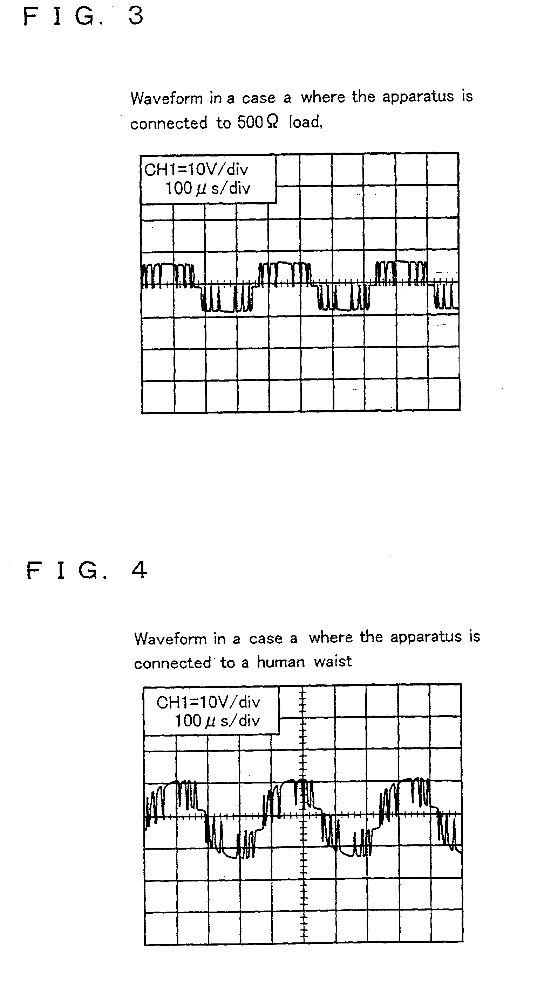

Fig.4 shows a waveform diagram of a stimulation signal which is actually applied to a human body. The stimulation signal shown here is defined by a rectangular wave pulse of recurring frequency 2.8kHz (T=357µSec). High frequency components are provided by varying the time width t2 in the range 10 to 60µSec. Fig. 3 shows a comparative waveform in the case a 500 Ω dummy resistor is connected as a load between the output electrodes 24. In that case, substantially the same waveform as that of the stimulation signal of Fig.2 is generated between the terminals of the dummy resistor. In contrast, Fig.4 shows a waveform in a case where the output electrodes 24 are attached to a human waist and then energized. In that case, the human body functions as if it were a capacitive element and the waveform of the rectangular wave pulse group S is distorted into a low frequency sinusoidal wave. Accordingly, an extremely soft feeling of stimulation is obtained as compared with rectangular waves of the same frequency and current. Furthermore, since the stimulation signals comprise high frequency components (obtained by switching the FETs 9 and 10) the therapeutic effects are enhanced.

In order that the stimulation signals exhibit a sinusoidal waveform such as the one illustrated in Fig.4, when applied to a human body, it is desirable that the rectangular pulses defining pulse groups S are alternately positive and negative, at intervals of period T. Furthermore, the time width t2 of a PWM pulse (on-pulse) is gradually increased during the first half of the pulse group S and then gradually decreased during the second half thereof. It should be noted that the on-pulse time width t2 can be varied by a time width varying means or a control sequence of the CPU 2. In addition, various waves such as triangular waves or pre-distorted waves can also be envisaged, thus enabling a unique stimulation feeling different from the one provided by sinusoidal waves.

When the above-mentioned stimulation signals are repeatedly applied to a human body, the latter may get accustomed to these signals and the therapeutic effects such as the removal or alleviation of pains are consequently lowered. In a further embodiment of the invention, the control sequence of the CPU 2 comprises pauses. The stimulation signal is temporarily stopped during the output of a stimulation pause setting signal from the CPU 2 to the output variable circuit 11. The stimulation pause setting signal indicates a stimulation pause period. Another way to prevent inurement to stimulation is to vary the amplitude of the stimulation signal. In response to the strong stimulation command signal output from the CPU 2, a rectangular pulse of larger amplitude A2 than the predetermined amplitude A1 is temporarily supplied by the output variable circuit 11 to the stimulus generator means 8. Thus, stronger stimulation signals i.e. pulse groups S' of greater amplitude are temporarily applied to the living body and the above-mentioned drawback is eliminated.

Since the stimulus generator means 8 modulates the pulse width of on-pulses within a rectangular pulse recurring at a given frequency, the resulting signal applied to the living body comprises components of higher frequency than said recurrence frequency. The human body having a capacitive impedance as mentioned above , the higher the frequency of a component of the signal, the lower the impedance at this frequency. It follows that the waveforms of the rectangular pulse groups are distorted as a whole, thereby generating softer feeling of stimulation as compared with the rectangular wave pulses of the same current and frequency. In addition, as each rectangular pulse group S includes signal components of higher frequency than the recurrence frequency of the rectangular wave pulse, extensive therapeutic effects can be obtained.

Moreover, the stimulus generator means 8 of the invention comprises the FETs 9 and 10 serving as switching means for on-off switching the rectangular wave pulse to thereby produce the rectangular wave pulse group S which includes signal components having higher frequency than the recurrence frequency. The CPU 2 serves as pulse width control means for supplying the FETs 9 and 10 with PWM digital signals (or +side PWM signals and -side PWM signals) for switching the FETs 9, 10.

To distort each rectangular wave pulse of the stimulation signal, the FETs 9, 10 (serving as switch means) are controlled with on-off PWM digital signals. There is therefore no need for conventional analogue circuit (such as the conventional D/A circuit 102 and the amplifier circuit 103) for obtaining the sinusoidal waveform. As a result, whilst conventional circuit for outputting sinusoidal waves would require dozens of components such as transistors, resistors and capacitors, the apparatus according to the present embodiment of the invention only requires a pair of FETs 9, 10, thus extremely simplifying the structure of the stimulus generator means 8 serving as the stimulation signal output circuit. Further, the pulse width modulation using the switch means exhibits very high power efficiency, as known in the domain of motor inverters.

The CPU 2 of the embodiment of the invention comprises strong stimulation controlling means for controlling the rectangular wave pulse amplitude: when strong stimulation mode is active, the rectangular wave pulse amplitude has an amplitude A2 which is greater than the predetermined amplitude A1. The strong stimulation controlling means enables therefore to apply stronger stimulation signals i.e. rectangular wave pulse groups S' of larger amplitude. This prevents a human body to get accustomed to the stimulation signals. Furthermore, the apparatus can be adapted to generate a rectangular wave pulse whose amplitude is randomly variable. Similarly, the pause period of the stimulation signal can also be made random. In both cases, a human body is more effectively prevented from getting accustomed to the stimulation signals. In addition, since the control sequence running on the CPU carries out the respective functions of said strong signal command means, stimulation pause command means as well as pulse width control means, the circuit has a less complicated structure than those known from the prior art.

The recurrence frequency of the rectangular wave pulse groups, the time width t1 of the rectangular wave pulse group S, the number and width t2 of the individual PWM pulses (on-pulses) may be chosen relatively freely according the various needs.

## Claims

1. Living body stimulating apparatus for applying an electric stimulus to a living body, said apparatus comprising electrodes to apply a stimulation signal to said living body and a stimulation signal generator (8,9,10), said stimulation signal generator being adapted to generate a stimulation signal from a periodic signal consisting of pulses of predetermined width (t1) recurring at a predetermined recurrence frequency (1/T), said stimulation signal comprising groups (S) of pulse width modulated pulses, which groups recur at said predetermined frequency, each group having a time width equal to said predetermined time width (t1), **characterized by** each group of pulses including at least two pulse width modulated pulses having a same sign.

2. Living body stimulating apparatus according to claim 1, **characterised in that** the stimulation signal generator is adapted to generate a stimulation signal comprising groups (S) of PWM pulses, the time width (t2) of a PWM pulse of a given group (S) gradually increasing during the first half of the time width of said given group (S) and gradually decreasing during the second half thereof.

3. Living body stimulating apparatus according to claim 2, **characterised in that** said stimulus generator comprises switching elements (9,10) for on-off switching said rectangular wave pulse signal, said apparatus further comprising pulse width control element (2) for switching the switching elements (9,10) with PWM digital signals

4. A living body stimulating apparatus according to claim 3, **characterised in that** said PWM digital signals are produced by a control sequence of a CPU (2).

5. A living body stimulating apparatus according to claim 2, **characterised in that** it comprises a strong stimulation controlling element generating a strong stimulation controlling signal (HAMMER) for temporarily outputting a rectangular wave pulse signal at a larger amplitude than at a predetermined one, which wave pulse signal is intended to constitute said periodic signal.

6. A living body stimulating apparatus according to claim 5, **characterised in that** said strong stimulation controlling signal is generated by a control sequence of the CPU (2).

7. A living body stimulating apparatus according to claim 1, **characterised in that** it comprises a signal pause setting element generating a signal pause setting signal (INTERVAL) for temporarily stopping the output of said stimulation signal.

8. A living body stimulating apparatus according to claim 7, **characterised in that** said the pause period of the stimulation signal is randomly varied.

9. A living body stimulating apparatus according to claim 7 or 8, **characterised in that** said stimulation pause setting signal is generated by a control sequence of a CPU (2) .

10. A living body stimulating apparatus according to claim 2, **characterised in that** the amplitude of said rectangular wave pulse is randomly varied.

## Patentansprüche

1. Reizvorrichtung für lebendige Körper zum Zuführen einer elektrischen Stimulanz an einen lebendigen Körper, wobei die Vorrichtung Elektroden zum Anlegen eines Stimulanzsignals an den lebendigen Körper sowie einen Stimulanzsignal-Generator umfasst, der so ausgebildet ist, dass er ein Stimulanzsignal aus einem periodischen Signal erzeugt, das aus Impulsen von vorbestimmter Länge (t1) besteht, die mit einer vorbestimmten Wiederholfrequenz (1/T) aufreten, wobei das Stimulanzsignal Gruppen (S) pulsbreiten-modulierter Impulse umfasst, welche Gruppen mit der vorbestimmten Frequenz auftreten, wobei jede Gruppe eine zeitliche Länge aufweist, die gleich der vorbestimmten Zeitlänge (t1) ist, **dadurch gekennzeichnet, dass** jede Gruppe von Impulsen wenigstens zwei pulsbreiten-modulierte Impulse mit gleichem Vorzeichen aufweist.

2. Reizvorrichtung für lebendige Körper nach Anspruch 1, **dadurch gekennzeichnet, dass** der Stimulanzsignal-Generator so ausgebildet ist, dass er ein Stimulanzsignal erzeugt, das Gruppen (S) pulsbreiten-modulierter (PWM) Impulse umfasst, wobei die zeitliche Länge (t2) eines PWM-Impulses einer beliebigen Gruppe (S) nach und nach während der ersten Hälfte der Zeitlänge der genannten beliebigen Gruppe (S) zunimmt und während der zweiten Hälfte derselben nach und nach abnimmt.

3. Reizvorrichtung für lebendige Körper nach Anspruch 2, **dadurch gekennzeichnet, dass** der Stimulanzsignal-Generator Schaltelemente (9, 10) zum An- und Abschalten des Rechteck-Impuls-Signals umfasst, wobei die Vorrichtung weiterhin ein Pulsweiten-Steuerelement (2) zum Schalten der Schaltelemente (9, 10) mit digitalen PWM-Signalen umfasst.

4. Reizvorrichtung für lebendige Körper nach Anspruch 3, **dadurch gekennzeichnet, dass** die digitalen PWM-Signale durch eine Steuersequenz einer CPU (2) erzeugt werden.

5. Reizvorrichtung für lebendige Körper nach Anspruch 2, **dadurch gekennzeichnet, dass** sie ein Stark-Stimulanz-Steuerelement umfasst, das ein Stark-Stimulanz-Steuersignal (HAMMER) zum zeitweisen Abgeben eines Rechteckwellen-Impuls-Signals mit größerer Amplitude als der Vorbestimmten erzeugt, wobei das Wellen-Impuls-Signal das periodische Signal darstellen soll.

6. Reizvorrichtung für lebendige Körper nach Anspruch 5, **dadurch gekennzeichnet, dass** das Stark-Stimulanz-Steuersignal durch eine Steuersequenz einer CPU (2) erzeugt wird.

7. Reizvorrichtung für lebendige Körper nach Anspruch 1, **dadurch gekennzeichnet, dass** sie ein Signalpausen-Stell-Element umfasst, das ein Signalpausen-Stell-Signal (INTERVAL) zum zeitweisen Unterbrechen der Abgabe des Stimulanzsignals erzeugt.

8. Reizvorrichtung für lebendige Körper nach Anspruch 7, **dadurch gekennzeichnet, dass** die Pausenperiode des Stimulanzsignals zufällig varriert wird.

9. Reizvorrichtung für lebendige Körper nach Anspruch 7 oder 8, **dadurch gekennzeichnet, dass** das Signalpausen-Stell-Signal durch eine Steuersequenz einer CPU (2) erzeugt wird.

10. Reizvorrichtung für lebendige Körper nach Anspruch 2, **dadurch gekennzeichnet, dass** die Amplitude des Rechteckwellen-Impulses zufällig varriert wird.

## Revendications

1. Appareil de stimulation de corps vivant permettant d'appliquer un stimulus électrique à un corps vivant, ledit appareil comprenant des électrodes pour appliquer un signal de stimulation audit corps vivant et un générateur de signal de stimulation (8, 9, 10), ledit générateur de signal de stimulation étant adapté pour produire un signal de stimulation à partir d'un signal périodique se composant d'impulsions de largeur prédéterminée (11) se reproduisant à une fréquence de récurrence prédéterminée (1/T), ledit signal de stimulation comprenant des groupes (S) d'impulsions modulées en largeur, lesquels groupes se reproduisent à ladite fréquence prédéterminée, chaque groupe ayant une largeur temporelle égale à ladite largeur temporelle prédéterminée (t1), **caractérisé en ce que** chaque groupe d'impulsions inclut au moins deux impulsions modulées en largeur ayant un même signe.

2. Appareil de stimulation de corps vivant selon la revendication 1, **caractérisé en ce que** le générateur de signal de stimulation est adapté pour produire un signal de stimulation comprenant des groupes (S) d'impulsions modulées en largeur, la largeur temporelle (t2) d'une impulsion modulée en largeur d'un groupe donné (S) augmentant petit à petit durant la première moitié de la largeur temporelle dudit groupe donné (S) et diminuant petit à petit durant la deuxième moitié de celle-ci.

3. Appareil de stimulation de corps vivant selon la revendication 2, **caractérisé en ce que** ledit générateur de stimulus comprend des éléments de commutation (9, 10) pour activer - désactiver ledit signal d'impulsion d'onde rectangulaire, ledit appareil comprenant de plus un élément de commande de largeur d'impulsion (2) pour la commutation des éléments de commutation (9, 10) avec des signaux numériques à modulation de largeur d'impulsions.

4. Appareil de stimulation de corps vivant selon la revendication 3, **caractérisé en ce que** lesdits signaux numériques à modulation de largeur d'impulsions sont produits par une séquence de commande d'une unité centrale de traitement (2).

5. Appareil de stimulation de corps vivant selon la revendication 2, **caractérisé en ce qu'**il comprend un élément de commande de stimulation énergique produisant un signal de commande de stimulation énergique (HAMMER) pour la délivrance de façon temporaire d'un signal d'impulsion d'onde rectangulaire à une amplitude plus grande que celle prédéterminée, lequel signal d'impulsion d'onde est prévu pour constituer ledit signal périodique.

6. Appareil de stimulation de corps vivant selon la revendication 5, **caractérisé en ce que** ledit signal de commande de stimulation énergique est produit par une séquence de commande de l'unité centrale de traitement (2).

7. Appareil de stimulation de corps vivant selon la revendication 1, **caractérisé en ce qu'**il comprend un élément de détermination de pause de signal produisant un signal de détermination de pause de signal (INTERVAL) pour stopper temporairement la sortie dudit signal de stimulation.

8. Appareil de stimulation de corps vivant selon la revendication 7, **caractérisé en ce que** ladite période de pause du signal de stimulation varie de façon aléatoire.

9. Appareil de stimulation de corps vivant selon la revendication 7 ou 8, **caractérisé en ce que** ledit signal de détermination de pause de stimulation est produit par une séquence de commande d'une unité centrale de traitement (2).

10. Appareil de stimulation de corps vivant selon la revendication 2, **caractérisé en ce que** l'amplitude de ladite impulsion d'onde rectangulaire varie de façon aléatoire.
